# EUROPEAN PATENT APPLICATION

(11) **EP 1 022 340 A1**
(43) Date of publication of application: **26.07.2000**
(21) Application number: 99200176.8
(22) Date of filing: 21.01.1999
(51) Int. Cl.: C12N 15/87, A61K 48/00

(54) **Stabilisation of compositions comprising non-viral nucleic acid transfer compositions**

(71) Applicant: OctoPlus B.V., 2333 CA Leiden (NL)
(72) Inventor: Hennink, Wilhelmus Everardus, 2743 CZ Waddinxveen (NL); Crommelin, Daniel Jan Anne, 3583 VP Utrecht (NL)
(74) Representative: Ottevangers, Sietse Ulbe

(57) **Abstract**

The present invention relates to a method for preparing a storage-stable and/or transportable cell-delivery nucleic acid composition comprising preparing an aqueous solution comprising said nucleic acid and, optionally, at least one cationic polymer. Further, the invention relates to a method for preparing a storage-stable and/or transportable cell-delivery nucleic acid composition free of cryopreservative and/or lyopreservative comprising preparing an aqueous solution comprising said nucleic acid and, optionally, at least one cationic polymer. In a further aspect, the invention relates to a method for preparing a cell-delivery nucleic acid composition comprising preparing an aqueous solution comprising said nucleic acid and, optionally, at least one cationic polymer and, before use, storing said solution for at least 2 weeks at a temperature between 0 and approximately the denaturation temperature of said nucleic acid in said solution, preferably at a temperature between 0 and approximately 40 °C. In addition, the storable products of these methods, as well as the use of the stabilising compounds are part of the present invention.

## Description

### FIELD OF THE INVENTION

The invention lies in the field of nucleic acid transfer, more particularly in the field of non-viral nucleic acid transfer.

### BACKGROUND OF THE INVENTION

Technology for the transfer of foreign genetic material into target cells has many potential applications, including therapeutic applications. Nucleic acid transfer technology can broadly be divided into two categories, i.e. transfer of nucleic acid using viral vector based nucleic acid delivery systems and non-viral vector based nucleic acid delivery systems. Both systems have their specific advantages and disadvantages. To overcome some of the specific disadvantages current research is among others directed toward combining elements from both categories. Non-viral nucleic acid delivery systems are attractive systems for nucleic acid delivery because they may be composed in vitro in an environment that can be completely controlled thus enabling a good control over the production process. Moreover, the safety aspects associated with non-viral nucleic acid delivery in for instance gene therapy are more easily controlled compared to viral vector based nucleic acid delivery. This is at least in part due to the synthetic nature of the transfer vehicle and the absence of viral components.

A problem with the current non-viral nucleic acid delivery systems is their relative instability. Previous studies using lipid based non-viral nucleic acid transfer systems have shown for example that lipid/DNA complexes readily form aggregates possessing reduced transfection efficiencies (Felgner et al, Ann. N.Y. Acad. Sci. 1995, **772:** 126-139; Gustafsson et al. Biochim. Biophys. Acta 1995, **1235:** 305-312).

The relative instability of non-viral nucleic acid delivery systems necessitates that, for instance in gene therapy applications, the delivery vehicle is prepared at the bed-side of the patient prior to the administration of the vehicle to the patient. The relative instability of the non-viral nucleic acid delivery vehicles has resulted in a generally accepted way of working with non-viral nucleic acid delivery systems, the way of working being that a non-viral nucleic acid delivery vehicle is prepared on the day of use and most of the times even within one hour before use. The requirement for preparation immediately prior to use introduces a step in the procedure that manufacturers of the nucleic acid delivery systems cannot control. In for instance pharmaceutical application of non-viral nucleic acid delivery vehicles, this lack of control over the final medicament for treatment of an individual is extremely undesired.

A prejudice against the stability of non-viral nucleic acid delivery vehicles has also led to the general practice of discarding any leftover nucleic acid delivery vehicles, as they were considered to deteriorate rapidly once prepared.

Attempts to minimise deterioration have been made and include, increasing the pH of the solution comprising the non-viral nucleic acid delivery vehicle, purification of more stable fractions from a solution comprising non-viral nucleic acid delivery vehicles and the addition of poly(ethylene glycol) moieties to the surface of the non-viral nucleic acid delivery vehicle (Caplen et al, Nat. Med. 1995, **1:** 39-46; Gao et al, Biochemistry 1996, **35:** 1027-1036; Hofland et al, Proc. Natl. Acad. Sci. 1996, **93:** 7305-7309; Hong et al, FEBS lett. 1997, **400:** 233-237).

While these approaches may have some effect on the stability of the delivery system, their application is not favoured because of the additional manipulations required rendering the production of the vehicles more complicated and less controllable. Moreover, the additional significant costs involved further disfavour their implementation in large scale productions. Lyophilisation of the non-viral nucleic acid delivery vehicle has also been attempted and although this seems to be effective also lyophilisation requires a bedside handling, i.e. the need to be rehydrated, and introduces additional manipulation and costs in the preparation of the non-viral nucleic acid delivery vehicle (Cherng et al, Pharm. Res. 1997, **14:** 1838-1841).

### SUMMARY OF THE INVENTION

The present invention provides a solution to the relative instability of non-viral nucleic acid delivery vehicles. The present invention allows non-viral nucleic acid delivery vehicles to be prepared in advance in a controlled environment, allowing them to be quality and safety tested prior to their use. The invention provides said solution without requiring additional handling of the prepared non-viral nucleic acid delivery vehicle and does not add significant costs. The invention further provides a means for the preparation of a non-viral nucleic acid delivery vehicle requiring no further manipulation prior to its use, for instance the administration to a patient. The invention further allows shipment and storage of the non-viral nucleic acid delivery system at ambient temperatures.

The present invention also provides means and methods for storing a nucleic acid in an aqueous solution.

### BRIEF DESCRIPTION OF THE DRAWINGS

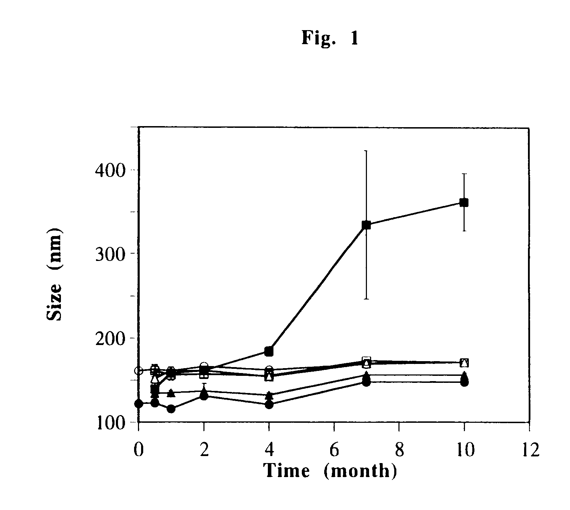

Figure 1: Size of polyplexes aged in 10% sucrose/aqueous solutions at 4°C (●), 20°C (▲) and 40°C (■) and in the freeze-dried state at 4°C (○), 20°C (△) and 40°C (□). The results are expressed as mean values ±S.D. of 3 experiments.

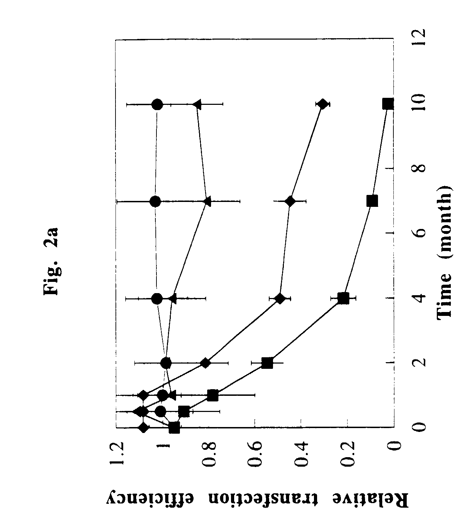

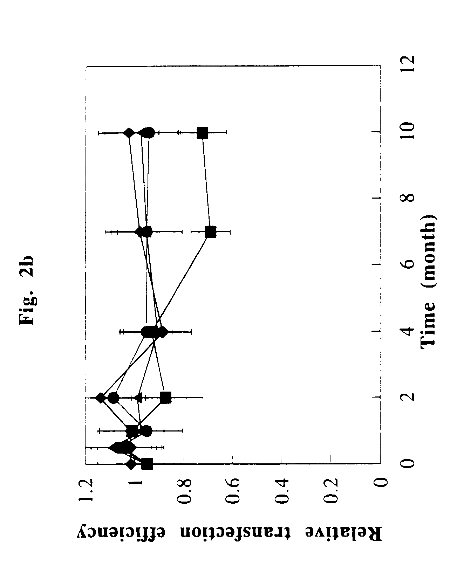

Figure 2: Relative transfection efficiency of aged polymer/plasmid complexes at 4°C (●), 20°C (▲) and 40°C (■) and naked plasmid at 40°C (◆). Aging was done in 10% sucrose/aqueous solutions (fig. 2a) or in freeze-dried form (10% sucrose, fig. 2b); naked plasmid was complexed with polymer just before the transfection experiment. The results are expressed as mean values ±S.D. of 3 experiments.

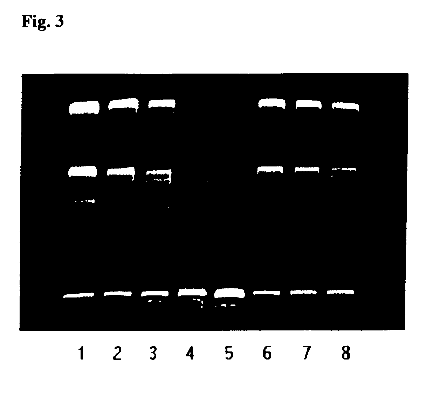

Figure 3: Agarose gel electrophoresis of plasmid. Naked plasmid (after storage for 10 months at 4°C) (lane 1); plasmid after dissociation of freshly prepared polyplexes (lane 2); plasmid after dissociation of aged polyplexes in sucrose/aqueous solutions after 10 months at 4°C (lane 3), 20°C (lane 4) and 40°C (lane 5) plasmid after dissociation aged polyplexes in freeze-dried form after 10 months at 4°C (lane 6), 20°C (lane 7) and 40°C (lane 8). Dissociation of the polyplexes was established by polyaspartic acid.

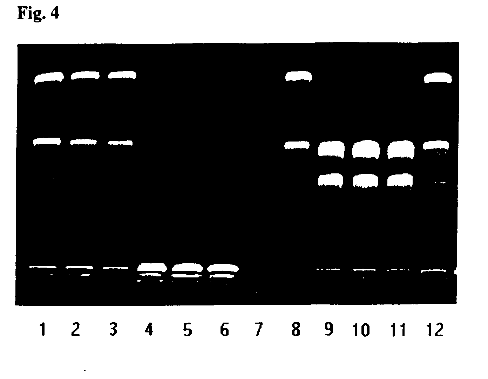

Figure 4: Agarose gel electrophoresis of plasmid. Naked plasmid (lane 1, 8 and 12); plasmid after dissociation of freshly prepared polyplexes (lane 2 (freshly prepared) and lane 3 (stored for 10 months at 4°C) ; plasmid after dissociation of polyplexes aged as an aqueous dispersion (Hepes, 20 mM, pH 7.4) at 40°C after 1 month (lane 4), 2 months (lane 5) and 4 months (lane 6). Naked plasmid aged as an aqueous dispersion (Hepes, 20 mM, pH 7.4) at 40°C for 1 month (lane 9), 2 months (lane 10) and 4 months (lane 11).

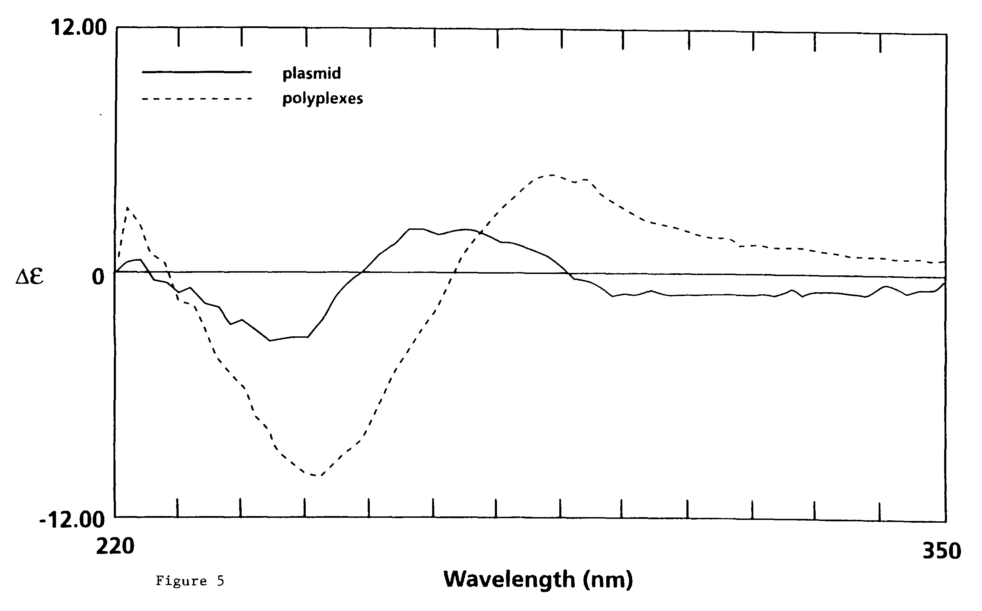

Figure 5: Typical CD spectra of free plasmid and freshly prepared polyplexes. The plasmid concentration was 13.3 µg/mL, the polymer/plasmid ratio was 3/1 (w/w).

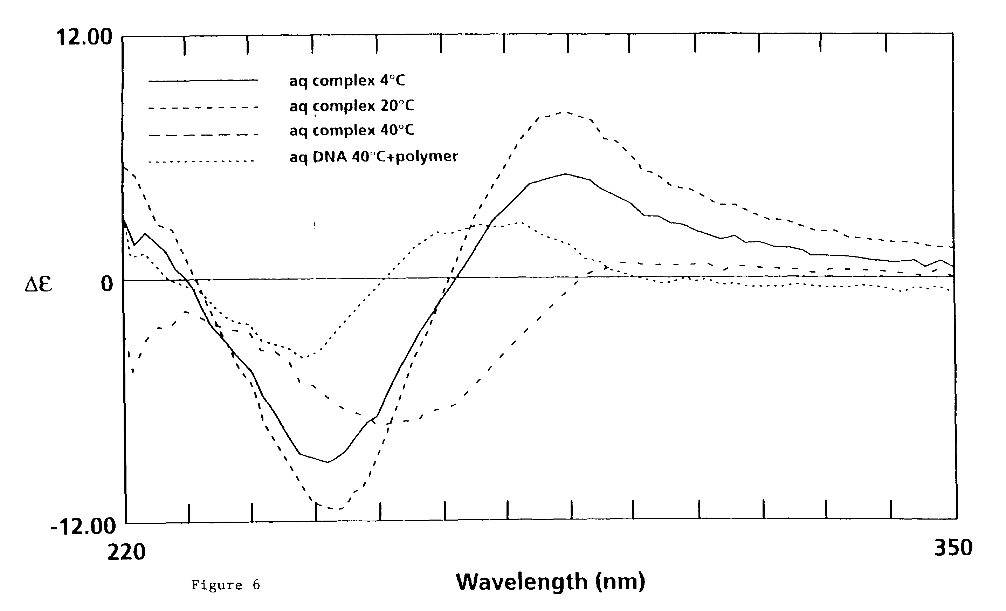

Figure 6: Typical CD spectra of polyplexes aged in 10% sucrose/aqueous solutions for 10 months at 4°C, 20°C and 40°C. The spectrum of aged plasmid (40°C, 10 months) and then complexed with polymer is also shown. The plasmid concentration was 13.3 µg/mL plasmid, the polymer/plasmid ratio was 3/1 (w/w).

### DETAILED DESCRIPTION OF THE INVENTION

In one aspect the present invention provides a method for preparing a storage-stable and/or transportable cell-delivery nucleic acid composition comprising preparing an aqueous solution comprising said nucleic acid and, optionally but preferably, at least one cationic polymer.

The invention further provides a method for preparing a storage-stable and/or transportable cell-delivery nucleic acid composition, which composition is essentially free from cryopreservative and/or lyopreservative comprising preparing an aqueous solution comprising said nucleic acid and, optionally but preferably, at least one cationic polymer. As used herein the term cryopreservative relates to a compound or a mixture of compounds which, when added to a cell-delivery nucleic acid composition, allows cryopreservation of said composition without substantial loss of cell-delivery activity upon freezing and thawing of said composition. As used herein the term lyopreservative relates a compound or a mixture of compounds which, when added to a cell-delivery nucleic acid composition, allows lyophilisation or freeze-drying of said composition without substantial loss of cell-delivery activity upon freeze-drying or lyophilisation and subsequent reconstitution with a suitable solvent.

The invention further provides a method for preparing a cell-delivery nucleic acid composition comprising preparing an aqueous solution comprising said nucleic acid and, optionally but preferably, at least one cationic polymer and, before use, storing said solution for at least 2 weeks at a temperature between 0 and approximately the denaturation temperature of said nucleic acid in said solution, preferably at a temperature between 0 and approximately 40 °C, and most preferably between 0 and 35°C.

In a preferred embodiment said aqueous solution is packaged before being stored. Preferably, said composition is stored at a temperature between 4 and approximately 25°C.

As used herein the term "cationic polymer" relates to water soluble polymers which in an aqueous solution have a net positive charge. Examples of such polymers are polyacrylates, polymethacrylates, polylysines, polyethyleneimines and polyorgano fosfazenes.

In a preferred embodiment at least one of said polymers is poly(2-dimethylamino)ethyl methacrylate (PDMAEMA) or a derivative or functional analogue thereof. This polymer has been described in, e.g., PCT/NL96/00416, the contents thereof being incorporated by reference for the polymer description.

Preferably, these water soluble cationic polymers, such as said PDMAEMA, have a weight average molecular weight M_{w} of at least 10,000 Da, preferably at least 50,000 Da, and most preferably at least 100,000 Da. The ratio of the weight average molecular weight to number average molecular weight M_{w}/Mₙ as common for radical polymerized polymers, such as between 5-20.

The concentration of the nucleic acid in said compositions is lower than the concentration giving rise to coaggulation, and is generally lower than 10 mg/ml, and preferably lies between 1 µg/ml and 10 mg/ml, and more preferably between 200 µg/ml and 500 µg/ml. The ratio polymer/nucleic acid lies in general between 0 and 6 (w/w), preferably between 1 and 4, most preferable between 1.5 and 3 (w/w).

In one aspect the invention provides a method for preparing a packaged, quality and safety tested aqueous solution comprising preparing a solution comprising a cell-delivery nucleic acid and, optionally but preferably, at least one cationic polymer capable of delivery of said nucleic acid to a cell, quality and safety testing said solution and packaging said solution in one or more containers suitable for packaging and storing said solution.

In another aspect the invention provides a use of an aqueous mixture of a cationic polymer and a cell-delivery nucleic acid, stored for at least 2 weeks at a temperature between 0 and approximately the denaturation temperature of said nucleic acid in said solution, preferably at a temperature between 0 and approximately 40 °C, and most preferably between 0 and 35°C in a package for storage, for the preparation of a cell-delivery nucleic acid pharmaceutical. Said use leads to a storage stable pharmaceutical comprising a cell-delivery nucleic acid. Said use leads to a storage stable pharmaceutical comprising a cell-delivery nucleic acid in active form.

In another aspect the invention provides a use of water to enhance the stability of nucleic acid delivery composition, which is optionally, but preferably, a cationic polymer based composition, at a temperature between 0 and approximately the denaturation temperature of said nucleic acid in said solution, preferably at a temperature between 0 and approximately 40 °C, and most preferably between 0 and 35°C. In yet another aspect the invention provides a use of a cationic polymer to enhance the stability of a composition comprising a cell-delivery nucleic acid at a temperature between 0 and approximately the denaturation temperature of said nucleic acid in said solution, preferably at a temperature between 0 and approximately 40 °C, and most preferably between 0 and 35°C.

In another aspect the invention provides the use of a cationic polymer for preparing a composition for storage and/or transportation of a cell-delivery nucleic acid comprising preparing an aqueous solution containing said nucleic acid and said cationic polymer.

The invention further provides a pharmaceutical comprising a composition of the invention.

In one embodiment the invention provides a packaged aqueous solution comprising a cell-delivery nucleic acid and, optionally but preferably, at least one cationic polymer capable of delivery of said nucleic acid to a cell, wherein said aqueous solution is contained in a package for storage and/or transportation of said solution.

In another embodiment the invention provides a packaged, quality and safety tested aqueous solution comprising a cell-delivery nucleic acid and at least one cationic polymer capable of delivery of said nucleic acid to a cell, wherein said aqueous solution is quality tested and safety tested and contained in a package for storage and/or transportation of said solution.

In another embodiment the invention provides a composition comprising a cell-delivery nucleic acid and at least one cationic polymer, wherein said composition has been stored for at least 2 weeks at a temperature between 0 and approximately the denaturation temperature of said nucleic acid in said solution, preferably at a temperature between 0 and approximately 40 °C, and most preferably between 0 and 35°C.

The invention further provides a method for preparing a packaged, quality and safety tested aqueous solution comprising a cell-delivery nucleic acid composition comprising preparing an aqueous solution comprising said nucleic acid and, optionally but preferably, at least one cationic polymer, performing quality and safety testing on a part of said solution and packaging, preferably in parallel with said quality and safety testing, other parts of said solution in one or more containers suitable for storage and/transport of said solution.

Quality testing of said composition may comprise for example assessing nucleic acid transfer capacity of said composition for instance on a marker cell line. Safety testing of said composition may comprise for example assessing toxicity parameters of said composition on marker cell lines or in test animals.

Furthermore, the invention is directed to an isolated nucleic acid in an aqueous solution, stored for at least 2 weeks at a temperature of between 4 and the denaturation temperature of said nucleic acid in said solution, preferably at a temperature between 0 and approximately 40 °C, and most preferably between 0 and 35°C. As can be seen from figure 2, naked nucleic acid has a higher stability at 40°C than a polyplex of the nucleic acid with cationic polymer.

In one aspect the invention provides a method for the preparation of a stable non-viral nucleic acid delivery composition comprising, mixing an aqueous solution comprising at least one nucleic acid with an aqueous solution comprising at least one cationic polymer and before using said composition, storing said composition for at least 2 weeks at . a temperature between 0 and approximately the denaturation temperature of said nucleic acid in said solution, preferably at a temperature between 0 and approximately 40 °C, and most preferably between 0 and 35°C.

Non-viral nucleic acid delivery compositions and/or aqueous solutions comprising nucleic acid, obtained through the present invention may be stored for at least two weeks, one month, two months up to 10 months or even longer. Stability may not necessarily mean stability of said nucleic acid but means stability of nucleic acid delivery capacity, and especially relates to enzymatic stability.

In a preferred embodiment the invention provides a method for the preparation of a pharmaceutical comprising a stable non-viral nucleic acid delivery composition wherein said preparation comprises, mixing an aqueous solution comprising at least one nucleic acid with an aqueous solution comprising at least one cationic polymer and before using said composition, storing said composition for at least 2 weeks at a temperature between 0 and approximately the denaturation temperature of said nucleic acid in said solution, preferably at a temperature between 0 and approximately 40 °C. In a preferred aspect of the invention said composition is stored at a temperature between 4 and approximately 25°C.

Preferably said nucleic acid delivery composition is prepared through the sterile mixing of a sterile aqueous solution comprising said nucleic acid with a sterile aqueous solution comprising said cationic polymer, whereupon all further procedures, for example the filling of vials, are performed under sterile conditions.

The concentration of nucleic acid and the concentration of cationic polymer in said nucleic acid delivery composition may vary with the specific application of said nucleic acid delivery composition. Typically, the concentration of cationic polymer and/or nucleic acid in said nucleic acid delivery composition varies with the cell type being targeted for nucleic acid transfer. The concentration of cationic polymer and/or the concentration of nucleic acid in said nucleic acid delivery composition may also be optimised to accommodate specific biological, structural, physical and/or chemical characteristics of the target cell(s) and/or target organ(s). For instance, delivery of a non-viral nucleic acid delivery composition to lung cells, for instance lung epithelial cells, will require penetration of the lung mucosa and/or the penetration deep into the alveoli.

Specific properties of nucleic acid delivery compositions and/or nucleic acid delivery vehicles contained therein may be composed through manipulation of the concentration of nucleic acid and/or the concentration of cationic polymer in said nucleic acid delivery composition. However, specific properties may also be added to said nucleic acid delivery composition in other ways. For instance, molecules preferably proteinaceous molecules, may be added to facilitate homing of said nucleic acid delivery vehicles to specific target cells. For instance but not limited to, the addition to said nucleic acid delivery vehicle of antigen binding parts of antibodies or the addition of receptor binding parts of extra-cellular signalling molecules such as growth factors or cytokines. Other properties that may be added to said nucleic acid delivery vehicles are for instance molecules promoting membrane disruption for instance of the endosomes, or molecules improving nuclear transfer of delivered nucleic acid.

In one aspect the invention provides the use of an aqueous mixture of a polymer and a nucleic acid, said mixture stored for at least 2 weeks at a temperature between 0 and approximately the denaturation temperature of said nucleic acid in said solution, preferably at a temperature between 0 and approximately 40 °C, for the preparation of a non-viral nucleic acid delivery pharmaceutical. Said mixture may be stored for at least two weeks, one month, two months up to 10 months or even longer.

In another aspect the invention provides the use of water to enhance the stability of a nucleic acid at a temperature between 0 and approximately the denaturation temperature of said nucleic acid in said solution, preferably at a temperature between 0 and approximately 40 °C.

In yet another aspect the invention provides the use of a cationic polymer to enhance the stability of an aqueous mixture comprising a nucleic acid, at a temperature between 0 and approximately the denaturation temperature of said nucleic acid in said solution, preferably at a temperature between 0 and approximately 40 °C.

In a preferred aspect of the invention said nucleic acid comprises a plasmid. Preferably said plasmid is present in supercoiled form. Preferably said preparation of said plasmid is essentially free of contaminating endotoxin and undesired protein.

In a preferred aspect of the invention an aqueous solution comprising a non-viral nucleic acid delivery composition comprises a buffer wherein said buffer buffers said solution between a pH of 6 and a pH of 8.

In a preferred aspect of the invention said aqueous solution further comprises a means for decreasing depurination and/or β-elimination of said nucleic acid.

In a preferred aspect of the invention said aqueous solution further comprises a means for decreasing free radical oxidation of said nucleic acid.

For a detailed description of PDMAEMA-nucleic acid complexes and their characteristics reference is made to the PhD thesis of Petra van de Wetering "Gene Delivery with cationic polymers; Synthesis, characterization and biological evaluation of poly(2-(dimethylamino)ethyl methacrylate) and related polymers" University of Utrecht (1999).

It is clear that as used herein the term "aqueous solution" also refers to an aqueous colloidal dispersion and/or an aqueous colloidal suspension. In particular when referring to a solution comprising a polymer, mixed with a nucleic acid or not.

It is clear that in a nucleic acid or a nucleic acid delivery vehicle of the invention said nucleic acid or said delivery vehicle may comprise one or more nucleic acid sequences physically linked or not.

### EXAMPLES

### MATERIALS AND METHODS

### Materials

pCMV-lacZ plasmid contains a bacterial lacZ gene preceded by a Nuclear Localization Signal under control of the CMV promoter (Bout, et al, Exp.Lung.Res., 1993, **19**: 193-202). The plasmid was isolated from *E. coli* and purified as described before (Cherng et al., Pharm. Res. 1996, **13**: 1038-1042). 2-(Dimethylamino)ethyl methacrylate (DMAEMA) was obtained from Fluka (Buchs, Switzerland). RPMI-1640 medium and DMEM (Dulbecco's modified Eagles medium) were obtained from Gibco, Breda, The Netherlands. Fetal Calf Serum (FCS) was purchased from Integron, Zaandam, The Netherlands. Cells were cultured in complete DMEM medium, which was prepared by supplementing plain DMEM with FCS (final concentration 5%), Hepes (final concentration 25 mM, pH 7.4), penicillin (final concentration 100 IU/mL), streptomycin (final concentration 100 µg/mL) and amphotericin B (final concentration 0.25 µg/mL). X-Gal (5-bromo-4-chloro-3-indoyl-β-galactopyranoside) was from Gibco, Breda, The Netherlands. Sucrose was obtained from Merck, Germany. Poly-L-aspartic acid (Mw.15,000-50,000) was obtained form Sigma Chemical Co., St. Louis, MO, USA. Agarose gel was from Hispanagar, Burgos, Spain. All other chemicals and reagents were of analytical grade. PDMAEMA (number average molecular weight (Mₙ) and weight average molecular weight (M_{w}) were 47 * 10³ and 280 * 10³ g/mol, respectively) was prepared by a radical polymerization of 2-(dimethylamino)ethyl methacrylate essentially as described previously (Cherng et al., Pharm. Res. 1996, **13**: 1038-1042).

### Preparation of PDMAEMA-plasmid Particles

PDMAEMA was dissolved in Hepes buffer (20 mM, pH 7.4) to a concentration of 300 µg/mL. The plasmid stock solution was diluted to a concentration of 400 µg/mL in the same buffer. Next, 100 µL plasmid solution was mixed with 500 µL aqueous solution of Hepes buffer (20 mM, pH 7.4) containing sucrose (20% w/v). Subsequently, 400 µL polymer solution was added and gently mixed for 5 s (Vortex Genie 2, Scientific Industries, INC., Bohemia, NT. USA). Thus, the final dispersions contained 20 mM Hepes, 0.12 mg/mL PDMAEMA, 0.04 mg/mL plasmid, and 10% sucrose. In case of freeze-drying, a 30 min incubation time at ambient temperature was observed and, subsequently, the formed complexes were frozen and freeze-dried (see next section).

### Freeze-Drying

Aliquots of PDMAEMA-plasmid dispersions of 1 mL were filled into 10 mL glass vials and frozen for 60 min by placing them on the shelf (pre-cooled by circulating silicone oil of -40°C) of a Leybold GT4 pilot-production freeze-dryer. The set pressure was 16 Pa which corresponds to the vapour pressure of ice at about -38°C. During the primary drying process, the temperature was kept below the collapse temperature of sucrose (-32°C) (Chang et al., Cryobiology 1992, **29**: 632-656). After 40 h, the shelf temperature was raised to +20°C with a rate of 10°C/h and kept at this temperature (under a pressure of 1 Pa) for 2 h. Finally, the vials were closed with a closing device under vacuum. For freeze-drying of plasmid DNA, 100 µL plasmid solution (400 µg/mL in Hepes buffer) was mixed with 500 µL aqueous Hepes buffer plus sucrose (20% w/v) and freeze-dried according to the same procedure.

### Aging of Polyplexes and Plasmid DNA

The freeze-dried and aqueous dispersions of polyplexes as well as the solutions of naked DNA were aged at 4°C, 20°C and 40°C for 10 months. Samples were taken at regular time points and evaluated for their physico-chemical properties (size, zeta-potential, DNA secondary and tertiary structure) and their transfection potential.

### Moisture Analysis

Residual moisture contents of the freeze-dried cakes were determined by the Karl-Fisher method using a Mitsubishi moisturemeter (model CA-05, Tokyo, Japan).

### Rehydration of the Freeze-Dried Cakes

After freeze-drying, the cakes of the polyplexes were reconstituted in 1 mL water and their size and zeta-potential were determined. For transfection studies, 7 mL RPMI-1640 medium was further added to this dispersion, resulting in a final concentration of 15 and 5 µg/mL for the polymer and plasmid, respectively. Freeze-dried naked plasmid was reconstituted in 600 µl water and 400 µl polymer solution (300 µg/ml) was added. This sample was used for size and zeta-potential measurements. For transfection, the same procedure as used for freeze-dried polyplexes was followed.

### Particle Size and Zeta-potential Measurements

The z-average particle size and polydispersity index (p.d.) of the PDMAEMA-plasmid particles were determined by dynamic light scattering (DLS) at 25°C with a Malvern 4700 system using 25 mW He-Ne laser (NEC Corp., Tokyo, Japan) and the automeasure version 3.2 software (Malvern Ltd., Malvern, UK). As a measure of the homogeneity of the colloid dispersion, the system reports a polydispersity index (p.d.). This index ranges from 0.0 for an entire homogeneous up to 1.0 for a completely inhomogeneous dispersion. For data analysis, the viscosity (0.8905 mPa.s) and refractive index (1.333) of water were used for the aqueous Hepes solution at 25°C. The refractive index and viscosity of a 10% sucrose solution were determined using a refractometer and by measuring the diffusion coefficient of a latex with known diameter (100 nm) essentially according to the method described by De Smidt and Crommelin, Int.J.Pharm. 1991, **77**: 261-264), respectively.

Zeta-potential measurements were conducted by determining the electrophoretic mobility at a temperature of 25°C with a Zetasizer 2000 (Malvern Ltd., Malvern, UK). For, viscosity and refractive index values: see above.

### Agarose Gel Electrophoresis

Freeze-dried naked plasmid and aqueous plasmid solutions aged at 40°C up to 10 months were analyzed by electrophoresis at 5.6 V/cm using a 0.7% agarose gel in Tris-acetate-EDTA buffer (pH 7.6). For each sample, 0.5 µg plasmid DNA was applied per slot. DNA was visualized by ethidium bromide (0.6 µg/mL in gel). To analyze plasmid complexed with polymer, 20 µl aliquot of the samples (0.8 µg plasmid and 2.4 µg polymer) was incubated with 5 µl of a polyaspartic acid solution (20 mg/mL PBS) to dissociate the polyplexes (Katayose et al., Bioconjugate Chem. 1997, **8**: 702-707). After 18 h incubation at ambient temperature, the samples were applied on a 0.7% agarose gel.

### Circular Dichroism (CD) Measurements

CD spectra were recorded from 220 to 350 nm at ambient temperature in quartz cells with a path length of 1 cm using a spectropolarimeter J-600 (Jasco, Tokyo, Japan). The scan rate was 20 nm/min and each measurement was the average of three repeated scans in steps of 2 nm. The values of λ and Δε are presented after subtraction of the CD scan of the appropriate buffer. The DNA concentration was 13.3 µg/ml(4* 10⁻⁵ M, based on number of DNA bases).

### Cell Culture and Transfection

For the gene transfer studies, COS-7 cells (cells of SV-40-transformed African green monkey kidney) were used essentially as described previously (Cherng et al., Pharm. Res. 1996, **13**: 1038-1042). The cells were seeded in a flat-bottom 96-well plate (1 x 10⁴ cells per well (0.38 cm²)) 24 h before transfection in complete DMEM. Thereafter, the PDMAEMA-plasmid complexes (volume 200 µl) were added to the cells and incubated for 1 h at 37°C and 5% CO₂. After removal of the transfection complexes, the cells were cultured for an additional 2 days in complete DMEM and then evaluated for transfection efficiency.

Expression of the pCMV-lacZ gene was established by incubations of fixed cells (0.25% glutaraldehyde; 5 min at 4°C) with a X-gal solution (0.8 mg/mL in phosphate buffer pH 7.4) together with 5 mM K₄Fe(CN)₆, 5 mM K₃Fe(CN)₆, 2 mM MgCl₂ for 24 h at 37°C. By using a light microscope, transfected cells were made visible as blue spots and were quantified by counting the number of blue spots in each well. Transfection values (relative transfection efficiency) were normalized to the number of transfected cells found after incubation of the cells with freshly prepared polymer/plasmid complexes (polymer/plasmid (w/w) ratio of 3, in Hepes buffer (20 mM, pH 7.4)).

### RESULTS

### Experimental set-up

Polyplexes were aged for a period of up to 10 months at three different temperatures (4°C, 20°C and 40°C) as aqueous dispersions as well as in the freeze-dried state. As a control naked plasmid was aged at 40°C. Sucrose was selected as a lyoprotectant since we demonstrated that this is an excellent compound to preserve the short term transfection potential of polyplexes (Cherng et al, Pharm.Res. 1997, **14**: 1837-1840). Moreover, 40°C was selected as highest aging temperature since at this temperature the matrix is still in its glassy state (Cherng et al, Pharm.Res. 1997, **14**: 1837-1840) (T_{g}=50°C). The aged polyplexes were evaluated for both their physico-chemical characteristics and their transfection potential. In a recent study it was demonstrated that no significant hydrolysis of the ester side chains of PDMAEMA occurred even at high temperature and extreme pH values, indicating that the polymer is stable (Wetering et al., Macromolecules, in press). Therefore, the attention was focused on changes which might occur in the secondary and tertiary structure of the plasmid both in its free form and complexed with polymer.

### Physico-Chemical Characteristics of the Aged Polyplexes

Dynamic light scattering measurements revealed that after rehydration of the cake, the size of the lyophilized polyplexes remained constant in time (diameter around 150 nm, fig. 1). This figure also shows that for the polyplexes aged as aqueous dispersions at 4°C and 20°C no change in particle size was observed either. On the other hand, the size of the polyplexes aged at 40°C gradually increased with time (from 150 to 350 nm after 10 months) suggesting that limited aggregation occurred. The size of the polyplexes obtained by complexing polymer with aged plasmid (lyophilized or aqueous) was not affected by aging and amounted to 150 nm. No changes in the charge of the polyplexes (zeta-potential 25-30 mV) were observed for the polyplexes aged under different conditions.

### Transfection Efficiency of Aged Polyplexes

Fig. 2a and Fig. 2b show the transfection potential of polyplexes aged in aqueous dispersions containing 10% sucrose and in the freeze-dried state, respectively. The polyplexes almost fully preserved their transfection potential after aging at 4 and 20°C (fig. 2a). But, polyplexes aged at 40°C were rather unstable and lost their transfection potential (half-life around 2 months). When naked plasmid DNA was aged in solution at 40°C and complexed with polymer just before the transfection experiment, a reduction in its transfection capability was observed as well. However, for naked plasmids loss of transfection efficiency was slower than for the polyplexes. Polyplexes and naked plasmid were also aged in aqueous media without sucrose at 40°C. Again, the plasmid in the polyplexes lost its transfection efficiency faster than naked plasmid: half-life 2 and 4 months, respectively.

After 10 month, no change in the morphology of the freeze-dried samples was observed by visual inspection. The moisture contents (0.5%) remained constant in time as well. As observed for the polyplexes aged in aqueous media at 4°C and 20°C, freeze-dried polyplexes aged at the same temperatures preserved their full transfection potential (fig. 2b). This figure also shows that, although some reduction in transfection efficiency was observed for the freeze-dried polyplexes aged at 40°C (20-30% reduction after 10 months), their stability was superior compared to polyplexes aged in aqueous media. Interestingly, the naked plasmid in freeze-dried form fully preserved its transfection potential, which suggests that plasmid complexed with polymer in freeze-dried form also lost its transfection potential faster than naked plasmid.

### DNA Tertiary Structure

Electrophoresis in agarose gels was used to study possible changes in the tertiary structure of plasmid DNA. Polyaspartic acid was used to dissociate the plasmid from the polymer in the polyplex. The plasmid used in this study consisted of a mixture of supercoiled and open circular DNA, with some traces of multimers (fig. 3, lane 1). The freshly prepared plasmid shows an identical pattern on agarose gel as free plasmid after the storage for 10 months at 4°C. When polyplexes were treated with polyaspartic acid shortly after their preparation, the same pattern was observed (lane 2), which indicates that complexation of polymer with plasmid does not lead to changes in the tertiary structure of the plasmid. Also, when the plasmid was liberated from lyophilized polyplexes with polyaspartic acid after 10 months of aging, the same electrophoretic pattern was observed as for freshly prepared naked plasmid (lanes 6-8). No large changes in the tertiary structure of the plasmid were observed either when the polyplexes were aged as an aqueous dispersion at 4°C (fig. 3, lane 3). However, supercoiled and open circular plasmid were hardly found in the polyplexes aged in aqueous media at 20°C and 40°C after 10 months and an intense band in and close to the application site was observed, particularly for the sample aged at 40°C (lane 4 and 5, fig. 3, respectively). Apparently, polyaspartic acid could not dissociate these polyplexes.

The tertiary structure of naked plasmid aged as an aqueous solution and in the lyophilized state was also analyzed. After 10 months of storage of naked plasmid in freeze-dried form at 40°C, the supercoiled and open circular form of the plasmid were still present (lane 8, fig. 3); the electrophoretic pattern was not significantly different from that of fresh plasmid. However, naked plasmid aged in an aqueous solution for 10 months at 40°C showed the presence of about 50% linear and 50% open-circular forms: supercoiled plasmid being virtually absent (results not shown).

To gain insight into the kinetics of the changes in the plasmid tertiary structure, polyplexes as well as naked plasmid were aged in aqueous solution at 4 and 40°C for 4 months. It was demonstrated that at 4°C no detectable changes in the structure occurred for the polyplexes and naked plasmid (fig. 4, lane 3 and 8, respectively). However, already after one month of aging at 40°C, no bands of supercoiled and open circular DNA were found in the polyplexes and most of the DNA hardly moved into the gel and stayed at the application site. Aging of naked plasmid in an aqueous solution at 40°C for one month showed that a major part of the DNA was in the open circular form, whereas only a minor part was still in the supercoiled form. Further aging resulted in the disappearance of the supercoiled form and the concomitant formation of open-circular and linear DNA, and an evidence of existence of oligomeric DNA or aggregates (fig. 4).

### DNA Secondary Structure

The CD spectrum of naked plasmid shows a positive peak with a maximum at about 274 nm and a negative peak with a minimum at 246 nm, which indicates a typical B-form DNA¹⁸⁻¹⁹ (fig. 5). Complexation with polymer resulted in a shift of both peaks to a higher wavelengths (290 and 254 nm, respectively), which suggests that a B to C transition had occurred (Arigita et al.; Weiskopf et al., Biopolymers 1977, **16:** 669-684). Moreover, complexation of plasmid and polymer is associated with an increase in the intensity of the negative peak. This can possibly be ascribed to formation of DNA helices which are oriented parallel to each other (so called ΨDNA (Ghirlando et al., Biochemistry 1992, **31**: 7110-7119). The freeze-dried polyplexes aged for 10 months at different temperatures (4°C, 20°C and 40°C) and the polyplexes stored as an aqueous dispersion at 4°C showed similar CD spectra as those obtained for freshly prepared polyplexes (see fig. 6, results of freeze-dried samples not shown). Even after 10 months, the changes in the CD spectrum of polyplexes aged as an aqueous dispersion at 20°C only concerned the Δε, not the peak maxima/minima (fig. 6). But, the CD spectrum of polyplexes aged as an aqueous dispersion at 40°C showed a gradual shift in the intensity and the wavelengths of both the maximum positive and the minimum negative peak (position after 10 months: 300 and 260 nm, respectively). Moreover, the change in Δε of the negative peak over time indicated that more ΨDNA is formed. Both observations suggest that the interaction between the polymer and plasmid became stronger in time.

The CD spectrum of naked plasmid aged in aqueous solution for 10 months was not significantly different from a fresh plasmid sample. Interestingly, when aged plasmid was complexed with polymer only a slight change in the CD spectrum was observed compared to the naked plasmid (slight shift of the position of both the maximum positive and the minimum negative peak to a higher wavelength; fig. 6).

## Claims

1. A method for preparing a storage-stable and/or transportable cell-delivery nucleic acid composition comprising preparing an aqueous solution comprising said nucleic acid and, optionally, at least one cationic polymer.

2. A method for preparing a storage-stable and/or transportable cell-delivery nucleic acid composition free of cryopreservative and/or lyopreservative comprising preparing an aqueous solution comprising said nucleic acid and, optionally, at least one cationic polymer.

3. A method for preparing a cell-delivery nucleic acid composition comprising preparing an aqueous solution comprising said nucleic acid and, optionally, at least one cationic polymer and, before use, storing said solution for at least 2 weeks at a temperature between 0 and approximately the denaturation temperature of said nucleic acid in said solution, preferably at a temperature between 0 and approximately 40 °C.

4. A method according to claim 3, wherein said aqueous solution is packaged before being stored.

5. A method according to claim 3 or claim 4, wherein said composition is stored at a temperature between 4 and approximately 25°C.

6. A method according to anyone of claims 1-5, wherein at least one of said polymers is poly(2-dimethylamino)ethyl methacrylate (PDMAEMA) or a derivative or functional analogue thereof.

7. A method according to claim 6 wherein said PDMAEMA has a weight average molecular weight of at least 10.000 Da, preferably at least 50,000 Da, and most preferably at least 100,000 Da.

8. A method according to anyone of the claims 1-7, wherein the concentration of said nucleic acid is lower than 10 mg/ml, preferably between 200 and 500 µg/ml.

9. A method according to anyone of claims 1-8, wherein at least one of said polymers is a poly organophosphazene.

10. Use of an aqueous mixture of a cationic polymer and a cell-delivery nucleic acid, stored for at least 2 weeks at a temperature between 0 and approximately the denaturation temperature of said nucleic acid in said solution, preferably at a temperature between 0 and approximately 40 °C in a package for storage, for the preparation of a cell-delivery nucleic acid pharmaceutical.

11. Use of water to enhance the stability of nucleic acid delivery composition, which is optionally cationic polymer based, at a temperature between 0 and approximately the denaturation temperature of said nucleic acid in said solution, preferably at a temperature between 0 and approximately 40 °C °C.

12. Use of a cationic polymer to enhance the stability of a composition comprising a cell-delivery nucleic acid at a temperature between 0 and approximately the denaturation temperature of said nucleic acid in said solution, preferably at a temperature between 0 and approximately 40 °C.

13. Use of a cationic polymer for preparing a composition for storage and/or transportation of a cell-delivery nucleic acid comprising preparing an aqueous solution containing said nucleic acid and said cationic polymer.

14. A pharmaceutical comprising a composition obtainable by a method according to anyone of claims 1-9 or by a use according to anyone of claims 10-13.

15. A packaged aqueous solution comprising a cell-delivery nucleic acid and, optionally, at least one cationic polymer capable of delivery of said nucleic acid to a cell, wherein said aqueous solution is contained in a package for storage and/or transportation of said solution.

16. A packaged quality and safety tested aqueous solution comprising a cell-delivery nucleic acid and, optionally, at least one cationic polymer capable of delivery of said nucleic acid to a cell, wherein said aqueous solution is quality tested and safety tested and contained in a package for storage and/or transportation of said solution.

17. A composition comprising a cell-delivery nucleic acid and at least one cationic polymer, wherein said composition has been stored for at least 2 weeks at a temperature between 0 and approximately the denaturation temperature of said nucleic acid in said solution, preferably at a temperature between 0 and approximately 40 °C.

18. A method for preparing a packaged, quality and safety tested aqueous solution comprising a cell-delivery nucleic acid composition comprising preparing an aqueous solution comprising said nucleic acid and, optionally, at least one cationic polymer, performing quality and safety testing on a part of said solution and packaging, preferably in parallel with said quality and safety testing, other parts of said solution in one or more containers suitable for storage and/transport of said solution.

19. An isolated nucleic acid in an aqueous solution, stored for at least 2 weeks at a temperature between 4 and the denaturation temperature of said nucleic acid in said solution, preferably at a temperature between 0 and approximately 40 °C.
